# EUROPEAN PATENT SPECIFICATION

(11) **EP 0 377 656 B1**
(45) Date of publication and mention of the grant of the patent: **27.04.1994**
(21) Application number: 88908513.0
(22) Date of filing: 24.08.1988
(51) Int. Cl.: A61M 29/02, A61M 25/00

(54) **CATHETER FOR BALLOON ANGIOPLASTY**
KATHETER FÜR BALLON-ANGIOPLASTIE
CATHETER POUR ANGIOPLASTIE A BALLON

(30) Priority: 24.08.1987 US 88264
(43) Date of publication of application: 18.07.1990
(73) Proprietor: PROGRESSIVE ANGIOPLASTY SYSTEMS, INC., Los Angeles, CA 90046 (US); HESS, Robert, Portola Valley, CA 90425 (US)
(72) Inventor: HESS, Robert, Portola Valley, CA 90425 (US)
(74) Representative: DIEHL GLAESER HILTL & PARTNER
(86) International application number: PCT/US88/02826
(87) International publication number: WO 89/01800

(56) References cited:
- EP-A- 0 182 689
- EP-A- 0 200 919
- WO-A-88/00844
- DE-A- 3 528 876
- US-A- 4 616 653
- US-A- 4 638 805
- US-A- 4 641 654
- US-A- 4 650 472
- US-A- 4 682 596
- US-A- 4 723 936
- US-A- 4 771 776
- US-A- 4 771 778

## Description

### BACKGROUND OF THE INVENTION

### Field of the Invention

This invention relates generally to surgical procedures employing a flexible catheter guide and more particularly to an apparatus for use in balloon angioplasty.

### Prior Art

Catheters require the capability of being pushed and flexibility in order to be effectively inserted into blood vessels and maneuvered through a vascular tree. Often a hollow spring wire shaft is used as a catheter, such catheter including a movable core wire.

A balloon may be used in conjunction with various catheter constructions such that the balloon may be inflated within the vessel, thereby opening blockages found therein. However, the combination of a hollow spring wire shaft and a movable core wire of variable properties has not been used in conjunction with a balloon.

U.S. Patent No. 4,276,874 discusses a balloon catheter including an elongated coil spring defining an inner passage, the spring being covered by silicone in the form of an extendable sheath, strain collar, and a balloon tip affixed to the strain relief collar. A portion of the elongated coil spring and sheath form a support structure which perceptibly elongates when excessive stretching force is applied to the support structure when moving the balloon through a body passage. Unfortunately, such a catheter is unsteerable and does not have variable flexibility.

Another device, disclosed in U.S. Patent No. 4,723,936, is a coil spring guide including a deflectable tip which comprises a coil spring covered by a sheath, a core wire within the coil spring extending the length of the coil spring, and a head member. At the proximal end of the coil spring guide a mechanism is provided to enable movement of the coil spring relative to the core wire. The core wire is eccentrically fixed to the back side of the head member and adjacent a lateral side thereof, thereby causing compession of the distal end spring coils and deflection thereof in a direction laterally from the side of the head member to which the core extension is fixed upon rearward movement of the core wire. Such a standard low profile catheter with a fixed wire system additionally does not offer a combined over-the-wire system nor variable column strength.

Still another device, disclosed in U.S. Patent No. 4,548,206, is directed to a catheter wire guide with a movable mandrel having a tapered tip which permits the flexibility of the distal tip of the wire guide to be varied. This device provides for a helically wound wire having an opening therethrough and mandrel positioned within the opening and longitudinally movable therein relative to the helically wound wire for varying the flexibility of the distal tip of the wire guide. The device is a diagnostic or wire placement device and not a balloon catheter suitable for angioplasty.

Yet another device, disclosed in US Patent 4 641 654, is directed to a steerable balloon dilation catheter having a guide wire adapted for limited rotation of the distal end of the guide wire. The device is primarily intended for dye injection and pressure measurement.

Although the foregoing devices include some advantageous features, they are limited in that they do not combine the following desirable features: a movable core means; variable stiffness of the shaft secondary to the movable core means and therefore variable trackability and pushability; a deflection mechanism for deflecting the tip of the catheter while still allowing torque and rotation; an ultra low profile, such that the device may be used in conjunction with a standard diagnostic coronary catheter rather than a guide catheter, referred to as a PTCA guiding catheter; the capability to transform from a fixed wire system to an over-the-wire system by removal of the movable core means; and the ability to insert either fiber optic angioscopes or laser fibers through the hollow portion of the catheter.

The foregoing illustrates limitations known to exist in present devices. Thus, it is apparent that it would be advantageous to provide an alternative device directed to overcoming one or more of the limitations set forth above. Accordingly, a suitable alternative is herein provided including features more fully disclosed hereinafter.

### SUMMARY OF THE INVENTION

One aspect of the present invention provides a catheter for balloon angioplasty comprising:
a flexible shaft having a hollow passage therein, said flexible shaft having a first proximal end which is open and a second distal end which is closed;
a core means movably and removably mounted within said passage, said core means having a first proximal end and a second distal end, said ends adjacent said first and second ends of said flexible shaft, respectively, said core means having a first flexible portion extending along its length from said first proximal end and a second flexible portion being of greater flexibility than said first flexible portion, said second flexible portion extending to said second distal end, said core means having an enlarged terminal knob at said second distal end thereof, said knob contacting said second distal end of said flexible shaft upon axial movement of said core means, further axial movement of said core means causing bending and buckling of said second flexible portion of said core means in turn bending said flexible shaft and causing angulation of said second distal end of said flexible shaft;
a flexible guide wire externally attached to the second distal end of said flexible shaft, said flexible guide wire axially extending away from said second distal end; and
an inflatable member having a non-inflatable sheath covering an external surface of said flexible shaft near the second distal end of said flexible shaft and further including means for inflating said inflatable member.

Another aspect of the instant invention provides a catheter for balloon angioplasty comprising:
a flexible shaft having a hollow passage therein, said flexible shaft having a first proximal end which is open and a second distal end which is partially open having a reduced cross-section;
a core means movably and removably mounted within said passage, said core means having a first proximal end and a second distal end, said ends adjacent said first and second ends of said flexible shaft, respectively, said core means having a first flexible portion extending along its length from said first proximal end and a second flexible portion being of greater flexibility than said first flexible portion, said second flexible portion extending to said second proximal end, said core means having an enlarged terminal knob at said second distal end thereof, said knob contacting said reduced cross-section of said second distal end of said flexible shaft upon axial movement of said core means, further axial movement of said core means causing bending and buckling of said second flexible portion of said core means bending said flexible shaft and causing angulation of said second distal end of said flexible shaft; and
a flexible guide wire attached to said terminal knob and axially extending away from said knob, said guide wire axially extensible through the opening in said second distal end of said flexible shaft.

Yet another aspect of the instant invention provides a catheter for balloon angioplasty comprising:
a flexible shaft having a sealed hollow passage defined therein and having a first proximal end and a second distal end, both said ends being open;
a core means movably, removably and substantially contained within said flexible shaft, said core means having a first proximate end and a second distal end and having a first flexible portion extending from said first proximate end and a second flexible portion terminating in said second distal end of said core means, said second flexible portion being of greater flexibility than said first flexible portion, said second flexible portion having a variable core strength along the length thereof from greater to lesser strength extending toward the second distal end of said core means to provide trackability, pushability and flexibility of said catheter;
a flexible guide wire fixed to and further axially extending from the second distal end of said core means; and
an inflatable member having a non-inflatable sheath covering an external surface of said flexible shaft near the second distal end of said flexible shaft and further including means for inflating said inflatable member.

Further aspects of the invention reside in providing a kit containing the catheters and other components sized to be used in conjunction with said catheters.

The foregoing and other aspects will become apparent from the following detailed description of the invention when considered in conjunction with the accompanying drawing. It is to be expressly understood, however, that the drawing is not intended as a definition of the invention but is for the purpose of illustration only.

### BRIEF DESCRIPTION OF THE DRAWING

Fig. 1 is a cross-sectional view illustrating a portion of a first embodiment of the catheter of the invention;
Fig. 2 is a second cross-sectional view illustrating the entire first embodiment of Fig. 1 of the invention;
Fig. 3 is a third cross-sectional view illustrating the first embodiment of Fig. 1 of the invention without an inflatable member and showing the core means withdrawn from the shaft;
Fig. 4 is a cross-sectional view illustrating a second embodiment of the invention;
Fig. 5 is a cross-sectional view illustrating a third embodiment of the invention including a power source;
Fig. 6 is a cross-sectional view illustrating a fourth embodiment of the invention;
Fig. 7 is a cross-sectional view of a fifth embodiment of the invention; and
Fig. 8 is a schematic view of a flow diagram of methods of using the present invention.

### DETAILED DESCRIPTION

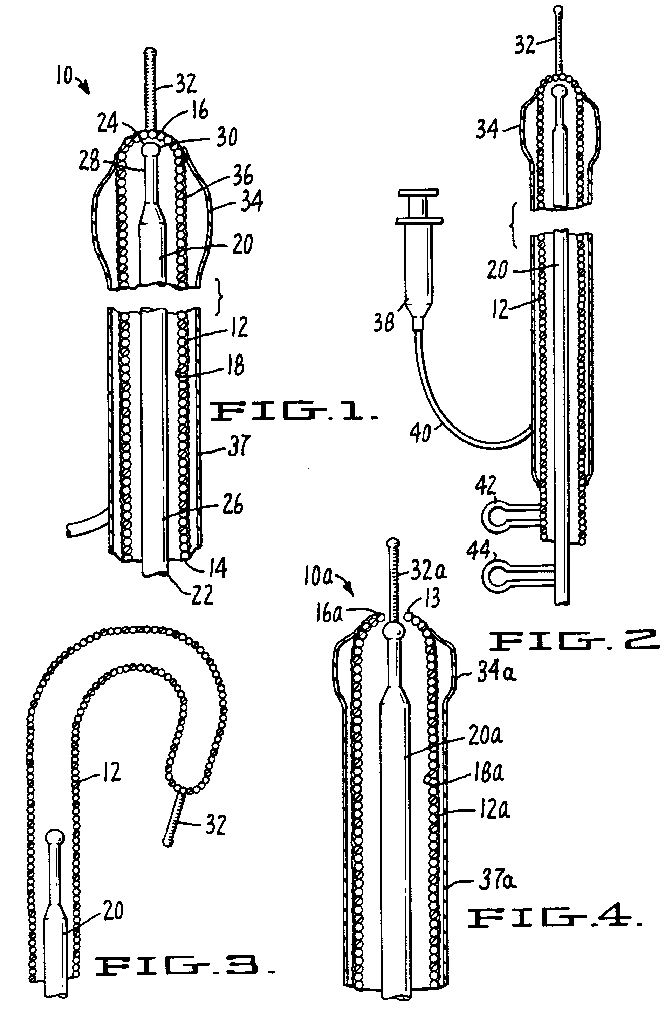

A catheter for balloon angioplasty, generally designated 10, and illustrated in Fig. 1, includes a flexible shaft 12 preferably formed of a suitable metal such as Type 304 Stainless Steel and having an outside diameter of from about 0.014 to about 0.035 inch (0.35 mm to about 0.88 mm). Shaft 12 has an open first or proximal end 14, a closed second or distal end 16, and defines a hollow passage 18 therein.

A flexible core means 20, preferably formed of a suitable metal such as Type 304 Stainless Steel has a diameter of from about 0.008 to about 0.030 inch (0.20 mm to about 0.75 mm). Core means 20 is movably and removably mounted within passage 18 and includes a first proximal end 22 and a second distal end 24 adjacent first and second ends 14, 16, respectively, of shaft 12. Core means 20 has a first portion 26 (extending from first end 22) which has a first flexibility and a second portion 28 (adjacent and terminating at second end 24) which is tapered to a smaller diameter than first portion 26 to provide a second flexibility, said second flexibility being greater than the aforesaid first flexibility. Core means 20 also includes an enlarged terminal knob 30 formed at second end 24.

A flexible guide wire 32, preferably formed of a suitable metal such as platinum to provide radiopacity and having a diameter of from about 0.008 to about 0.018 inch (0.20 mm to about 0.45 mm), may be fixed securely by suitable means such as adhesives, welding or brazing, to second end 16 of shaft 12. Guide wire 32 is of a greater flexibility than the aforesaid second flexibility.

An inflatable member such as an angioplasty balloon 34 of polymeric material is attached or affixed to second end 16 of shaft 12 to substantially cover an external surface 36 of shaft 12 in such a manner that second end 16 of shaft 12 and guide wire 32 are free of such covering. Such angioplasty balloons are wellknown in the art and can be formed in one piece along with a non-inflatable sheath or as a separate component and attached to a non-inflatable shaft. A separate component may allow the use of dissimilar materials between the non-inflatable sheath and the balloon. As seen in Figs. 1, 2 and 4-7 the balloons 34, 34a and 34b are formed as a one-piece component with non-inflatable sheath 37, 37a and 37b. It is understood that shaft 12 is sealed with respect to passage 18, such as by use of a plastic covering, so that balloon 34 may be inflated. A suitable inflation means 38 (see Fig. 2) may be connected via conduit 40 to inflate balloon 34. A finger loop 42 is suitably connected to shaft 12, and a corresponding finger loop 44 is similarly connected to core means 20.

Catheter 10 is constructed of a spring wire shaft 12 with a movable and removable core means 20. This feature is desirable in that it allows variable column strength of shaft 12 and variable flexibility which is controlled by the operator by either advancing or withdrawing movable core means 20. If stiffness of the shaft is required to push through tight lesions, core means 20 is advanced, and the entire catheter 10 becomes stiffer and has greater ability to be pushed. If extreme flexibility is required, as may be the case in navigating through extremely tortuous vessels, the core means 20 of catheter 10 may be withdrawn; see Fig. 3. Core means 20 may be withdrawn partially or totally, thereby increasing the flexibility of catheter 10 from the distal portion to the proximal portion, depending upon how far movable core means 20 is withdrawn. Because shaft 12 of catheter 10 consists of spring wire rather than polymers, shaft 12, even without core means 20, has a fair degree of column strength, yet has extreme flexibility. During any given angioplasty procedure, either extreme flexibility or column strength, or both, may be required; therefore, during any procedure, core means 20 may be pulled back and advanced according to the desires of the operator. As an additional feature, core means 20 may be replaced with a second core means of greater or lesser flexibility. Thus, a "fine-tuning" of column strength is available to the operator.

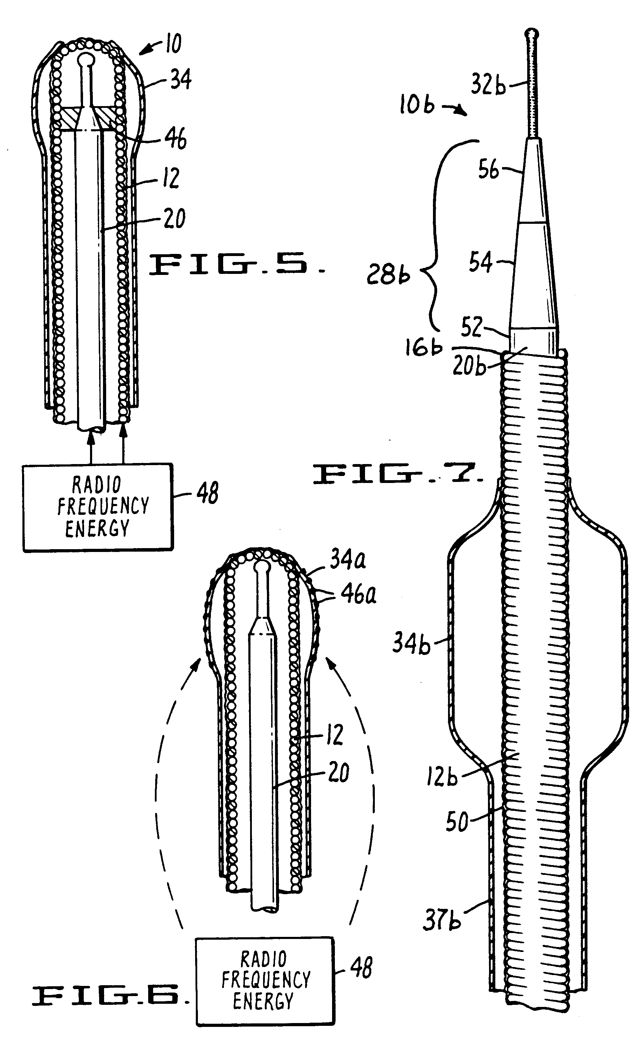

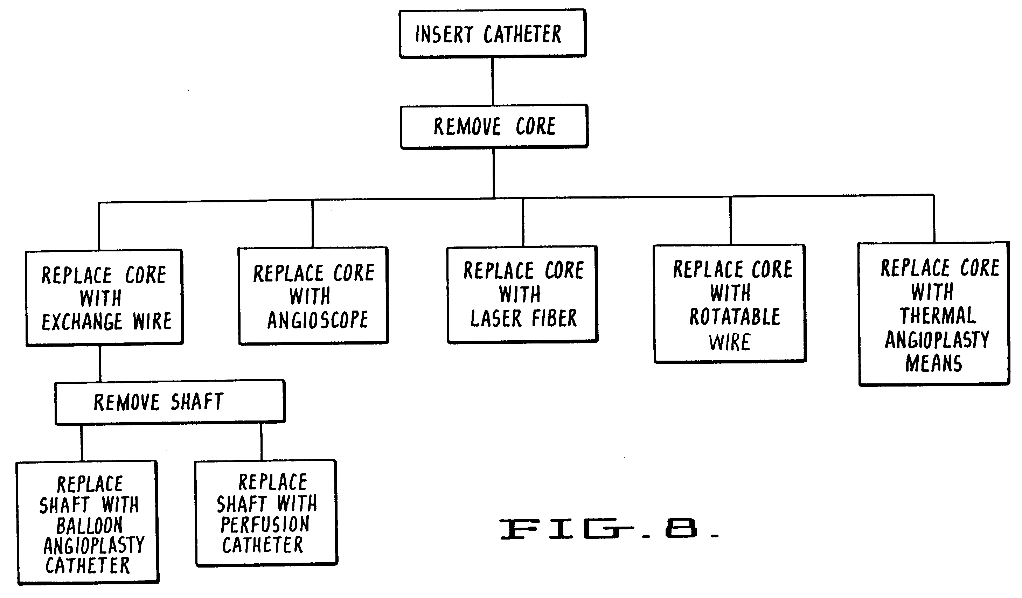

A modification (third embodiment) of the shaft of Fig. 1 is illustrated in Fig. 5 and includes a ferrite material 46 provided within shaft 12. Means 48 provides a radio frequency signal to be received by ferrite material 46 using shaft 12 and core means 20 as conductors, thus heating catheter 10. Alternatively, another modification (fourth embodiment) is illustrated in Fig. 6 and may include the ferrite material 46a impregnated within a portion of the inflatable member 34a. Similarly, means 48 provides the aforesaid radio frequency signal to be received by ferrite material 46a.

Another modification (second embodiment) of the shaft is illustrated in Fig. 4 wherein shaft 12a includes an aperture 13 formed in second end 16a. Core means 20a is also modified in that flexible guide wire 32a is fixedly attached to knob 30a. Balloon 34a and finger loops 42, 44 (described above in connection with shaft 12 and core means 20 illustrated in Fig. 2) may also be associated with the combination of shaft 12a and core means 20a of Fig. 4.

The unique construction of catheter 10 (Fig. 1) and catheter 10a (Fig. 4) allows for both tip deflection and torquing of leading guide wire 32. In the first, the shaft 12 of catheter 10 is closed at the distal end. As core means 20 pushes axially forward and is advanced into contact with closed end 16, catheter 10 is deflected. This deflection is a result of the fact that core means 20 is more flexible in portion 28 than in portion 26. This increased flexibility of the distal portion of core means 20 (as opposed to the proximal portion) is the result of either tapering core means 20 so that it is thinner distally than proximally, or by creating a joint therein such that an elbow effect is created at the joint -- when the catheter is pushed forward, the elbow bends deflecting the end 24 of core means 20 and the end 16 of shaft 12 as well, because shaft 12 is a flexible spring wire. The greater the force applied to core means 20, the greater the extent of angulation of catheter 10. The radius of angulation of catheter 10 is controlled by the location of the taper or by the position of the elbow joint. The closer the elbow joint to end 24, or the shorter the taper at end 24, the smaller the radius of angulation and vice versa. Angulation of greater than 120° can be obtained using these criteria.

The end of core means 20 is blunted by terminal knob 30 in order to provide a large surface bearing area between the core means 20 and the closed end 16. In Fig. 1, a very flexible guide wire 32 of about 1-7 cm in length and 0.20 mm to about 0.45 mm in diameter may be fixedly connected to end 16 of shaft 12. This "fixed wire", as connected to the distal portion of shaft 12, allows the entire catheter 10 to be advanced through the arteries with minimal trauma. Guide wire 32 can be deflected by deflecting end 16 of shaft 12 and can be torqued by torquing shaft 12 of catheter 10.

The similar but slightly modified and steerable catheter 10a is illustrated in Fig. 4. In this variation, distal end 16a of shaft 12a is not entirely closed but includes aperture 13. Core means 20a is similar to core means 20, except that the highly flexible terminal guide wire 32 is preferably welded to knob 30a. In this system, core means 20a is withdrawn or advanced to vary the shaft stiffness, as explained above. The main difference between the variations is that flexible guide wire 32 is connected directly to the core means, rather than to the shaft. As core means 20a is advanced, guide wire 32 advances so that it protrudes through aperture 13 in end 16a of shaft 12a. However, the diameter of knob 30a is larger than the diameter of aperture 13 at the position of the weld between guide wire 32 and core means 20a. Therefore, in order to deflect catheter 10a, the operator axially advances core means 20a so that knob 30a pushes against the distal end 16a of shaft 12a. In this manner, the core means is deflected due to the taper in core means 20a. This deflects both shaft 12a of catheter 10a and guide wire 32 which protrudes from end 16a. Further, if the operator requires torque on guide wire 32, the operator rotates the proximal portion of core means 20a thereby transmitting the torque through core means 20a to the distal guide wire 32, and thus torquing the guide wire. Therefore, this system allows the operator to have both tip angulation and the ability to torque through an ultra low profile system with variable shaft flexibility.

Fig. 7 illustrates a fifth embodiment of the invention, shown generally at 10b, having a movable and removable core means 20 of variable strength, as will be discussed further. Core means 20b is contained within flexible shaft 12b having a second distal end 16b. Flexible shaft 12b is preferably a coated wire wound member (see coating 50), which preferably constitutes an inner lumen surrounded by balloon 34b of polymeric material. Guide wire 32b extends from the end of core means 20b. Core means 20b is described as having variable core strength because, similar to the earlier-described embodiments, it has a first flexible portion 26b extending from the first proximate end of the core means 20b and a second flexible portion 28b terminating in the second distal end of the core means 20b. Second flexible portion 28b preferably comprises a series of tapered cross-sections 52, 54 and 56 of different diameter, thereby exhibiting a variable strength when subjected to bending. These cross-sections may be tapered, as shown, or varied by using progressively smaller uniform cross-sections connected to each other in a stepped fashion. Other equivalent structures are within the scope of the invention. It can be seen that the axial position of the core means 20b relative to the second distal end of the flexible shaft 12b will determine the over-all trackability, pushability, flexibility and steerability of the distal end of the catheter. The further the second flexible portion 28b (tapered cross-sections 52, 54 and 56) of core means 20b extends beyond second distal end 16b of the shaft 12b, the larger the cross-section of the core means in the vicinity of balloon 34b and therefore the greater the stiffness of the distal end of the catheter. The flexibility of the distal end of the catheter is determined by a lesser cross-section in the vicinity of balloon 34b which is accomplished by retraction of the second flexible portion 28b with respect to second distal end 16b. Movement of said second flexible portion 28b of said core means axially relative to said second distal end 16b of said flexible shaft again determines the flexibility, pushability, trackability and steerability of the catheter. Extension of the second flexible portion 28b relative to the second distal end 16b of said flexible shaft increases pushability of the catheter and decreases flexibility and trackability, retraction of the second flexible portion 28b from such extended position increasing flexibility and trackability of the catheter with an accompanying decrease in pushability.

Because catheter 10 is constructed with a movable core means 20, shaft 12 becomes hollow when core means 20 is removed. In Fig. 4, aperture 13 (in end 16a of shaft 12a) can be used in several settings. This also applies to Fig. 7. If the operator proceeding with this system comes upon an unexpected closure of the artery necessitating the insertion of a long exchange wire so that a "bail out" catheter may be placed or a larger or smaller balloon can be inserted, then the operator may remove core means 20a of catheter 10a and insert a long exchange wire through the hollow passage 18a of shaft 12a, the long exchange wire protruding through aperture 13 in end 16a of shaft 12a; due to the absence of knob 30a, shaft 12a may be removed, and a larger or smaller balloon or "bail-out" catheter may be placed over it. This is a unique feature of this system in that no other angulating, torquable catheter is able to provide the capabilities of both a fixed wire and an over-the-wire system in the same catheter.

It can be seen that all of the above also applies to the structure of Fig. 4 and Fig. 7 wherein like components have like reference numerals. For the purpose of the following discussion, it is understood that the embodiments of Figs. 4 and 7 are both open at their distal ends and allow similar procedures. For the sake of illustration, some attempt will be made in describing both embodiments by referring to the reference numerals in both Figs. 4 and 7.

The new procedures of coronary angioscopy and laser angioplasty may have significant use as adjuncts to balloon angioplasty. The catheter of this invention has significant applicability to both angioscopy and laser angioplasty. If the operator is using catheter 10a of Fig. 4 (or 10b of Fig. 7) and then decides to perform coronary angioscopy, the following steps can be undertaken. First, catheter 10a (or catheter 10b) is advanced to the location of interest using the standard technique. Second, core means 20a (or core means 20b) of catheter 10a (or catheter 10b) is withdrawn, and the operator then inserts an appropriately-sized, highly-flexible angioscope into the shaft of the catheter and advances it to aperture 13. Therefore, this system uniquely provides the ability to perform coronary angioscopy during balloon angioplasty. By advancing the coronary angioscope through hollow passage 18a, the angioscope can be advanced without its actually coming into contact with the arterial wall. That is, the angioscope is always enclosed while within the shaft 12a (or shaft 12b) of catheter 10a (or catheter 10b). This is a significant improvement over current methods of coronary angioscopy which expose the tip of the angioscope to the wall of the artery, thereby allowing the potential for abrasion and dissection of the arterial wall. Another hazard of current methods is of protrusion of the sharp tip of the angioscope through the plastic at bend regions. The metallic structure of catheter 10a (or catheter 10b) prevents the protrusion from occurring in this system. The flushing of blood may then be performed through the guiding catheter (with or without the balloon inflated) to allow a clear field in which to view with the angioscope.

Similarly, if laser angioplasty is performed, the laser fiber (connected to virtually any laser source) can be advanced through hollow passage 18a of catheter 10a up to and, if necessary, protruding through aperture 13. This, again, allows advancement of the laser fiber without contacting the arterial wall, thus minimizing the potential for abrasion. This function is not easily performed with conventional polymer catheters since the tip of the laser fiber can be quite sharp and may protrude through the plastic at bend regions. The metallic structure of catheter 10a (or catheter 10b) prevents protrusion with this system.

Fig. 8 shows a schematic flow diagram of alternative methods using other well-known components sized to fit, with respect to the catheters of the instant invention, in surgical techniques. These components will continue to be described.

Balloon 34a (or balloon 34b) is placed directly over shaft 12a (or shaft 12b) in this balloon angioplasty system. Balloon 34a (or balloon 34b) is constructed of polymer, the polymer being connected and attached or affixed to distal end 16a (or end 16b) of shaft 12a (or shaft 12b). Balloon 34a (or balloon 34b) is somewhat proximal to end 16a (or end 16b); then the polymer extends back to the proximal portion of catheter 10a (or catheter 10b). The proximal portion of balloon 34a (or balloon 34b) is connected to the balloon inflation apparatus, as shown in Fig. 2. Balloon 34a (or balloon 34b) can be composed of any one of a variety of standard materials (polyethylene, polyvinylchloride) or may be made of new ultra-thin materials, such as the DuPont product PET. Since balloon 34a (or balloon 34b) is placed directly over shaft 12a (or shaft 12b) and the balloon material can be very low profile, this catheter has an extremely low profile, only slightly above that of shaft 12a (or shaft 12b). Shaft 12a (or shaft 12b) can have a variable size (depending upon what is desired) and can range from about 0.014 inch (0.35 mm) to any upper size desired. The incorporation of novel polymer technology is also possible. In this system, some catheters could be composed of available polymers and strengthened with thin strands of impregnated fibers. This would allow very high burst strengths (in excess of 15 atmospheres) yet not create a high profile. This would allow for high pressure inflations with a very low profile catheter system. Because of the extremely low profile, this system may be inserted through conventional diagnostic. catheters rather than the special PTCA guide catheters currently used in all angioplasty procedures. The diagnostic catheters have handling characteristics superior to the PTCA guide catheters and are also smaller in diameter and less traumatic to the coronary arteries. The use of diagnostic catheters, therefore, provides a significant advantage over available systems.

The optionally provided pistol grip handles provided by loops 42, 44 connected to the proximal portion of shaft 12, 12a and core means 20, 20a allow very fine control of the core means and the shaft and manipulation by only one hand (Fig. 2). This, therefore, provides significant advantages over currently available systems. The pistol grip handles allow simple advancement or withdrawal of the entire catheter system; individual advancement or withdrawal of the core means and shaft; torque of the shaft, the core means, or both; advancement or withdrawal of the core means to vary shaft flexibility and the shaft's ability to be pushed; and core means advancement so that tip angulation can be achieved through the mechanisms described above. This is a significant advantage over currently available systems.

A modified version of catheter 10a could also be useful in balloon valvuplasty. The modifications would incorporate a larger balloon (3-8 cm in length, 12-25 mm in diameter). Shaft 12a of Fig. 4 would be employed. These modifications would allow tip steerability for retrograde crossing of the aortic valve (no current valvuplasty system has steerability), and a movable core means for variable flexibility and column strength. (Great flexibility is required for navigating up tortuous, peripheral vessels to reach the valve, yet column strength is required to move across the narrowed valve orifice.) Once the valve is crossed, the core means can be removed, allowing pressure monitoring through the hollow shaft. (Distal pressure monitoring during valvuplasty is not possible with conventional systems, and the spring wire is sufficiently flexible and atraumatic to sit in the ventricle without the core means and tip wire.) The low profile of the shaft with its fiber-strengthened balloon allows for an over-all low profile.

A significant modification can be achieved using catheter 10 or 10a, or virtually any conventional balloon angioplasty catheter. There is scientific evidence to support the notion that heating the artery to a temperature greater than some critical temperature, currently thought to be about 70°C., may ameliorate the restenosis found in 25-30% of patients following balloon angioplasty. Restenosis is considered to be the single most significant complication of balloon angioplasty, and any device which could ameliorate this complication would have enormous clinical impact. Several potential solutions to this problem are proposed; all are related to methods of heating the balloon. One solution (Fig. 5) involves placing a small amount of ferrite material 46 at the distal end of core means 20 in the balloon portion of catheter 10. This ferrite material is ferromagnetic and is capable of being heated when coupled to an appropriately tuned radio frequency energy source. Radio frequency energy in the megahertz or microwave portion of the electromagnetic spectrum is transmitted down shaft 12 of angioplasty catheter 10 using core means 20 and shaft 12 as conducting material. This energy then couples to the ferrite material 46 and generates intense localized heating. Heat from ferrite material 46 heats the fluid used to inflate balloon 34, providing a balloon heated to greater than 70°C. This balloon could be used for coronary or peripheral arterial inflation, and by virtue of the temperature achieved is capable of killing smooth muscle cells in the wall of the artery. These smooth muscle cells are responsible for restenosis following angioplasty, and it is claimed that the thermal energy from the balloon catheter would kill the progenitor cells responsible for restenosis. Another solution (Fig. 6) involves the impregnation of the balloon material itself with the ferromagnetic material 46. The radio frequency energy is then delivered from outside the body and is coupled into the ferromagnetic material 46 in balloon 34. That is, the ferromagnetic material in balloon 34 acts as an antenna for the radio frequency energy. This generates intense heating (greater than 70°C.) of balloon 34 and kills the smooth muscle cells, thereby preventing restenosis. Other suitable means of heating a balloon will yield equally effective results.

Described above is a system for producing a small central lumen in a totally occluded artery which could then be followed by balloon angioplasty. If an artery is totally occluded it is difficult to place the balloon into the occlusion so that inflation can commence. It is proposed that the above-described catheter 10a can be used to solve this problem. Catheter 10a can be advanced to the blockage. Then core means 20a can be removed, leaving behind the hollow shaft 12a. Through passage 10a in shaft 12a a specially fabricated wire which protrudes through aperture 13 can be placed. The proximal portion of the new central wire is connected to a rotating mechanism which is powered by either batteries or standard electric current. The rotating mechanism rotates from about 10,000 to about 250,000 rpm, thereby causing the central wire to rotate at the same frequency. This rotating wire will act as a drill and burrow a small central channel through the totally occluded artery. Once this has occurred, the catheter is advanced through the blockage such that the balloon is then placed within the blockage, the balloon being capable of dilating the blockage. This procedure can be applied to sequential recanalization and balloon dilation of totally occluded arteries.

## Claims

1. A catheter (10) for balloon angioplasty comprising:
a flexible shaft (12) having a hollow passage (18) therein, said flexible shaft having a first proximal end (14) which is open and a second distal end (16) which is closed; and
an inflatable member (34) having a non-inflatable sheath (37) covering an external surface of said flexible shaft near the second distal end of said flexible shaft and further including means (38) for inflating said inflatable member,
said catheter being characterized by further comprising:
a core means (20) movably and removably mounted within said passage, said core means having a first proximal end (22) and a second distal end (24), said end adjacent said first and second ends of said flexible shaft respectively, said core means having a first flexible portion (26) extending along its length from said first proximal end and a second flexible portion (28) being of greater flexibility than said first flexible portion, said second flexible portion extending to said second distal end, said core means having an enlarged terminal knob (30) at said second distal end thereof, said knob contacting said second distal end of said flexible shaft upon axial movement of said core means, further axial movement of said core means causing bending and buckling of said second flexible portion of said core means in turn bending said flexible shaft and causing angulation of said second distal end of said flexible shaft; and
a flexible guide wire (32) externally attached to the second distal end of said flexible shaft, said flexible guide wire axially extending away from said second distal end.

2. A catheter (10a) for balloon angioplasty comprising:
a flexible shaft (12a) having a hollow passage (18a) therein, said flexible shaft having a first proximal end which is open and a second distal end (16a) which is partially open having a reduced cross-section; and
an inflatable member (34a) having a non-inflatable sheath (37a) covering an external surface of said flexible shaft near the second distal end of said flexible shaft and further including means (38) for inflating said inflatable member,
said catheter being characterized by further comprising:
a core means (20a) movably and removably mounted within said passage, said core means having a first proximal end and a second distal end, said end adjacent said first and second ends of said flexible shaft respectively, said core means having a first flexible portion extending along its length from said first proximal end and a second flexible portion being of greater flexibility than said first flexible portion, said second flexible portion extending to said second distal end, said core means having an enlarged terminal knob at said second distal end thereof, said knob contacting said reduced cross-section of said second distal end of said flexible shaft upon axial movement of said core means, further axial movement of said core means causing bending and buckling of said second flexible portion of said core means bending said flexible shaft and causing angulation of said second distal end of said flexible shaft; and
a flexible guide wire (32a) attached to said terminal knob and axially extending away from said knob, said guide wire axially extensible through the opening in said second distal end of said flexible shaft.

3. A catheter as in Claims 1 or 2, further including ferrite material (46) within said flexible shaft near said second distal end of said flexible shaft and including means (48) for transmitting radio frequency energy through said flexible shaft to said ferrite material to cause heating of said ferrite material to heat said second distal end of said flexible shaft.

4. A catheter as in claims 1 to 3, in the form of a kit, further comprising:
components in the form of an exchange wire, an angioscope, a laser fiber, a rotatable wire, and a thermal angioplasty means for thermal oblation and remodeling of tissue, all of said components sized to be insertable within said flexible shaft upon removal of said core means; and
a different balloon angioplasty catheter and a perfusion catheter, said balloon angioplasty catheter and said perfusion catheter sized to be inserted over said exchange wire.

5. A catheter (10b) for balloon angioplasty comprising:
a flexible shaft (12b) having a sealed hollow passage defined therein and having a first proximal end and a second distal end, both said ends being open; and
an inflatable member (34b) having a non-inflatable sheath covering (37b) an external surface of said flexible shaft near the second distal end of said flexible shaft and further including means (38) for inflating said inflatable member.
said catheter being characterized by further comprising:
a core means (20b) movably, removably and substantially contained within said flexible shaft, said core means having a first proximate end and a second distal end and having a first flexible portion extending from said first proximate end and a second flexible portion (28b) terminating in said second distal end of said core means, said second flexible portion being of greater flexibility than said first flexible portion, said second flexible portion having a variable core strength along the length thereof from greater to lesser strength extending toward the second distal end of said core means to provide trackability, pushability and flexibility of said catheter;
a flexible guide wire (32b) fixed to and further axially extending from the second distal end of said core means.

6. A catheter as in Claim 5 wherein said second flexible portion of said core means comprises a series of tapered sections 56, 54.

7. A catheter as in claims 5 or 6, in the form of a kit, further comprising:
components in the form of an exchange wire, an angioscope, a laser fiber, a rotatable wire, and a thermal angioplasty means for thermal oblation and remodeling of tissue, all of said components sized to be insertable within said flexible shaft upon removal of said core means; and
a different balloon angioplasty catheter and a perfusion catheter, said balloon angioplasty catheter and said perfusion catheter sized to be inserted over said exchange wire.

## Patentansprüche

1. Katheter (10) für Ballon-Angioplastie mit:
einem flexiblen Schaft (12), in dessen Innerem ein Hohldurchgang (18) ausgebildet ist, wobei der flexible Schaft ein offenes, erstes, proximales Ende (14) und ein geschlossenes, zweites, distales Ende (16) aufweist, und
einem aufblasbaren Teil (34) mit einer nicht aufblasbaren Hülle (37), die eine äußere Oberfläche des flexiblen Schafts nahe des zweiten, distalen Endes des flexiblen Schafts bedeckt und desweiteren Mittel (38) zum Aufblasen des aufblasbaren Teils umfaßt,
wobei der Katheter dadurch gekennzeichnet ist, daß er desweiteren aufweist:
einen Kern (20), der innerhalb des Durchgangs bewegbar und entfernbar angeordnet ist, wobei der Kern ein erstes, proximales Ende (22) und ein zweites, distales Ende (24) aufweist, die benachbart zu dem ersten Ende bzw. dem zweiten Ende des flexiblen Schafts belegen sind, und der Kern einen ersten flexiblen Abschnitt (26), der sich in Längsrichtung von dem ersten, proximalen Ende aus erstreckt, und einen zweiten flexiblen Abschnitt (28) aufweist, der von größerer Flexibilität ist, als der erste flexible Abschnitt und sich zu dem zweiten, distalen Ende hin erstreckt, wobei der Kern im Endbereich seines zweiten, distalen Endes mit einem vergrößerten Kopf (30) ausgebildet ist, der das zweite, distale Ende des flexiblen Schafts kontaktiert, wenn der Kern in axialer Richtung bewegt wird, wobei eine weitergehende axiale Bewegung des Kerns zu einem Abbiegen und Wölben des zweiten flexiblen Abschnitts des Kerns führt, wodurch der flexible Schaft abgebogen und ein Abwinkeln des zweiten, distalen Endes des flexiblen Schafts bewirkt wird, sowie
einen flexiblen Führungsdraht (32), der von außen an dem zweiten, distalen Ende des flexiblen Schafts befestigt ist, wobei sich der flexible Führungsdraht axial von dem zweiten, distalen Ende weg erstreckt.

2. Katheter (10a) für Ballon-Angioplastie mit:
einem flexiblen Schaft (12a), in dessen Innerem ein Hohldurchgang (18a) ausgebildet ist, wobei der flexible Schaft ein offenes, erstes proximales Ende und ein zweites, teilweise offenes, distales Ende (16a) mit verkleinertem Querschnitt aufweist, und
einem aufblasbaren Teil (34a) mit einer nicht aufblasbaren Hülle (37a), die eine äußere Oberfläche des flexiblen Schafts nahe des zweiten, distalen Endes des flexiblen Schafts bedeckt und desweiteren Mittel (38) zum Aufblasen des aufblasbaren Teils aufweist,
wobei der Katheter dadurch gekennzeichnet ist, daß er desweiteren aufweist:
einen Kern (20a), der innerhalb des Durchgangs bewegbar und entfernbar angeordnet ist, wobei der Kern ein erstes, proximales Ende und ein zweites, distales Ende aufweist, die benachbart zu dem ersten Ende bzw. dem zweiten Ende des flexiblen Schafts belegen sind, und der Kern einen ersten flexiblen Abschnitt, der sich in Längsrichtung von dem ersten, proximalen Ende aus erstreckt, und einen zweiten flexiblen Abschnitt aufweist, der von größerer Flexibilität ist, als der erste flexible Abschnitt und sich zu dem zweiten, distalen Ende hin erstreckt, wobei der Kern im Endbereich seines zweiten, distalen Endes mit einem vergrößerten Kopf ausgebildet ist, der den Abschnitt des zweiten, distalen Endes des flexiblen Schafts mit verkleinertem Querschnitt kontaktiert, wenn der Kern in axialer Richtung bewegt wird, wobei eine weitergehende axiale Bewegung des Kerns zu einem Abbiegen und Wölben des zweiten, flexiblen Abschnitts des Kerns führt, wodurch der flexible Schaft abgebogen und ein Abwinkeln des zweiten, distalen Endes des flexiblen Schafts bewirkt wird, sowie
einen flexiblen Führungsdraht (32a), der an dem im Endbereich vorgesehen Kopf befestigt ist und sich von diesem axial weg erstreckt, wobei der flexible Führungsdraht axial durch die Öffnung im zweiten, distalen Ende des flexiblen Schafts verlängerbar ist.

3. Katheter nach Anspruch 1 oder 2, der desweiteren Ferritmaterial (46) innerhalb des flexiblen Schafts nahe dem zweiten, distalen Ende des flexiblen Schafts aufweist und Mittel (48) enthält zur Übertragung von Hochfrequenzenergie durch den flexiblen Schaft zu dem Ferritmaterial, um ein Erwärmen des Ferritmaterials und damit ein Erwärmen des zweiten, distalen Endes des flexiblen Schafts zu bewirken.

4. Katheter nach Anspruch 1 bis 3, in Form eines Gerätesatzes, der desweiteren aufweist:
Komponenten in Form eines Austauschdrahtes, eines Angioskops, einer Laserfaser, eines drehbaren Drahtes, und eines Mittels zur Durchführung von Thermoangioplastie zur thermischen Abflachung und Remodellierung von Gewebe, wobei alle diese Komponenten so dimensioniert sind, daß sie nach Entfernen des Kerns in den flexiblen Schaft einführbar sind, und
einen weiteren Katheter für Ballon-Angioplastie und einen Perfusionskatheter, die so dimensioniert sind, daß sie über den Austauschdraht einführbar sind.

5. Katheter (10b) zur Ballon-Angioplastie mit:
einem flexiblen Schaft (12b), welcher einen abgedichteten Hohldurchgang im Innern und ein erstes, proximales Ende und ein zweites, distales Ende aufweist, wobei die Enden offen sind, und
einem aufblasbaren Teil (34b) mit einer nicht aufblasbaren Hülle (37b), die eine äußere Oberfläche des flexiblen Schafts nahe des zweiten, distalen Endes des flexiblen Schafts bedeckt und desweiteren Mittel (38) zum Aufblasen des aufblasbaren Teils aufweist,
wobei der Katheter dadurch gekennzeichnet ist, daß er desweiteren aufweist:
einen beweglichen, entfernbaren und im wesentlichen in dem flexiblen Schaft aufgenommenen Kern (20b), der ein erstes, proximales Ende und ein zweites, distales Ende sowie einen ersten, flexiblen Abschnitt, der sich von dem ersten, proximalen Ende erstreckt, sowie einen zweiten flexiblen Abschnitt (28b), der in dem zweiten, distalen Ende des Kerns endet, aufweist, wobei der zweite flexible Abschnitt von größerer Flexibilität ist als der erste flexible Abschnitt und der zweite flexible Abschnitt eine variable Kernstärke in seiner Längsertreckung von einer größeren zu einer kleineren Stärke in Richtung des zweiten distalen Ende des Kerns aufweist, um dem Katheter Nachführbarkeit, Verschiebbarkeit und Flexibilität zu verleihen, und
einen flexiblen Führungsdraht (32b), der an dem zweiten, distalen Ende des Kerns befestigt ist und sich von diesem axial weg erstreckt.

6. Katheter nach Anspruch 5, bei dem der zweite flexible Abschnitt des Kerns eine Reihe von abgeschrägten Bereichen (56, 54) aufweist.

7. Katheter nach Anspruch 5 oder 6, in der Form eines Gerätesatzes, der desweiteren aufweist:
Komponenten in Form eines Austauschdrahtes, eines Angioskops, einer Laserfaser, eines drehbaren Drahts und eines Mittels zur Durchführung von Thermoangioplastie zur thermischen Abflachung und Remodellierung von Gewebe, wobei alle diese Komponenten so dimensioniert sind, daß sie nach Entfernung des Kerns in den flexiblen Schaft einführbar sind, und
einen weiteren Katheter für Ballon-Angioplastie und einen Perfusionskatheter, die so dimensioniert sind, daß sie über den Austauschdraht einführbar sind.

## Revendications

1. Cathéter (10) pour angioplastie à ballon comprenant :
- un axe flexible (12) présentant un passage creux (18), ledit axe flexible ayant une première extrémité proximale (14) qui est ouverte et une seconde extrémité distale (16) qui est fermée ; et
- un élément gonflable (34) ayant un manchon non gonflable (37) couvrant une surface externe dudit axe flexible au voisinage de la seconde extrémité distale dudit axe flexible et comprenant en outre des moyens (38) de gonflage dudit élément gonflable,
ledit cathéter étant caractérisé en ce qu'il comprend en outre :
- un moyen formant noyau (20) monté mobile et amovible à l'intérieur dudit passage, ledit moyen formant noyau ayant une première extrémité proximale (22) et une seconde extrémité distale (24), lesdites extrémités étant respectivement voisines desdites première et seconde extrémités dudit axe flexible, ledit moyen formant noyau ayant une première portion flexible (26) s'étendant depuis ladite première extrémité proximale, et une seconde portion flexible (28) d'une plus grande flexibilité que ladite première portion flexible, ladite seconde portion flexible s'étendant vers ladite seconde extrémité distale, ledit moyen formant noyau ayant un bouton terminal agrandi (30) à ladite seconde extrémité distale dudit moyen, ledit bouton venant en contact avec ladite seconde extrémité distale dudit axe flexible lors du mouvement axial dudit élément formant noyau, la poursuite du mouvement axial dudit moyen formant noyau provoquant la flexion et le flambage de ladite seconde portion flexible dudit moyen formant noyau, courbant alors ledit axe flexible et provoquant la formation d'un angle dans ladite seconde extrémité distale dudit axe flexible ; et
- un fil de guidage flexible (32) attaché à l'extérieur de la seconde extrémité distale dudit axe flexible, ledit fil de guidage flexible s'étendant axialement à partir de ladite seconde extrémité distale et à l'écart de celle-ci.

2. Cathéter (10a) pour angioplastie à ballon, comprenant :
- un axe flexible (12a) présentant un passage creux (18a), ledit axe flexible ayant une première extrémité proximale qui est ouverte et une seconde extrémité distale (16a) qui est partiellement ouverte et présente une section transversale réduite ; et
- un élément gonflable (34a) ayant un manchon non gonflable (37a) couvrant une surface externe dudit axe flexible au voisinage de la seconde extrémité distale dudit axe flexible et comprenant en outre des moyens (38) de gonflage dudit élément gonflable,
ledit cathéter étant caractérisé en ce qu'il comprend en outre :
- un moyen formant noyau (20a) monté mobile et amovible à l'intérieur dudit passage, ledit moyen formant noyau ayant une première extrémité proximale et une seconde extrémité distale, lesdites extrémités étant respectivement voisines desdites première et seconde extrémités dudit axe flexible, ledit moyen formant noyau ayant une première portion flexible s'étendant depuis ladite première extrémité proximale, et une seconde portion flexible d'une plus grande flexibilité que ladite première portion flexible, ladite seconde portion flexible s'étendant vers ladite seconde extrémité distale, ledit moyen formant noyau ayant un bouton terminal agrandi à ladite seconde extrémité distale dudit moyen, ledit bouton venant en contact avec ladite section transversale réduite de ladite seconde extrémité distale dudit axe flexible lors du mouvement axial dudit élément formant noyau, la poursuite du mouvement axial dudit moyen formant noyau provoquant la flexion et le flambage de ladite seconde portion flexible dudit moyen formant noyau, courbant alors ledit axe flexible et provoquant la formation d'un angle dans ladite seconde extrémité distale dudit axe flexible ; et
un fil de guidage flexible (32a) attaché audit bouton terminal et s'étendant axialement depuis ledit bouton et à l'écart de celui-ci, ledit fil de guidage étant axialement extensible au travers de l'ouverture de ladite seconde extrémité distale dudit axe flexible.

3. Cathéter selon la revendication 1 ou 2, comprenant en outre du matériau ferrite (46) à l'intérieur dudit axe flexible au voisinage de ladite seconde extrémité distale dudit axe flexible et comprenant des moyens (48) pour transmettre de l'énergie de fréquence radio au travers dudit axe flexible vers ledit matériau ferrite pour provoquer le chauffage dudit matériau ferrite et chauffer ladite seconde extrémité distale dudit axe flexible.

4. Cathéter selon les revendications 1 à 3, sous la forme d'une unité, comprenant en outre :
- des composants sous la forme d'un fil d'échange, d'un angioscope, d'une fibre laser, d'un fil rotatif, et de moyens d'angioplastie thermiques pour l'ablation thermique et le remodelage de tissus, tous lesdits composants étant dimensionnés de façon à pouvoir être insérés à l'intérieur dudit axe flexible lors de l'enlèvement dudit moyen formant noyau ; et
- un cathéter d'angioplastie à ballon différent et un cathéter de perfusion, ledit cathéter d'angioplastie à ballon et ledit cathéter de perfusion étant dimensionnés pour pouvoir être insérés sur ledit fil d'échange.

5. Cathéter (10b) pour angioplastie à ballon, comprenant :
- un axe flexible (12b) ayant un passage creux fermé de façon étanche et ayant une première extrémité proximale et une seconde extrémité distale, lesdites extrémités étant toutes deux ouvertes ; et
- un élément gonflable (34b) ayant un manchon non gonflable (37b) couvrant une surface externe dudit axe flexible au voisinage de la seconde extrémité distale dudit axe flexible et comprenant en outre des moyens (38) de gonflage dudit élément gonflable,
ledit cathéter étant caractérisé en ce qu'il comprend en outre :
- un moyen formant noyau (20b) monté de façon mobile et amovible et sensiblement contenu à l'intérieur dudit axe flexible, ledit moyen formant noyau ayant une première extrémité proximale et une seconde extrémité distale et ayant une première portion flexible s'étendant à partir de ladite première extrémité proximale et une seconde portion flexible (28b) se terminant dans ladite seconde extrémité distale dudit moyen formant noyau, ladite seconde portion flexible ayant une plus grande flexibilité que ladite première portion flexible, ladite seconde portion flexible ayant une résistance de noyau variable le long de sa longueur passant d'une résistance plus grande à une résistance plus faible tandis que l'on va vers la seconde extrémité distale dudit moyen formant noyau, pour offrir au cathéter la possibilité d'être pisté, poussé et fléchi ;
- un fil de guidage flexible (32b) fixé à ladite seconde extrémité distale dudit moyen formant noyau et s'étendant davantage axialement à partir de celle-ci.

6. Cathéter selon la revendication 5, dans lequel ladite seconde portion flexible dudit moyen formant noyau est constituée d'une série de sections effilées (56, 54).

7. Cathéter selon les revendications 5 ou 6, sous la forme d'un ensemble, comprenant en outre :
- des composants sous la forme d'un fil d'échange, d'un angioscope, d'une fibre laser, d'un fil rotatif, et de moyens d'angioplastie thermiques pour l'ablation thermique et le remodelage de tissus, tous lesdits composants étant dimensionnés de façon à pouvoir être insérés à l'intérieur dudit axe flexible lors de l'enlèvement dudit moyen formant noyau ; et
- un cathéter d'angioplastie à ballon différent et un cathéter de perfusion, ledit cathéter d'angioplastie à ballon et ledit cathéter de perfusion étant dimensionnés pour pouvoir être insérés sur ledit fil d'échange.
